# EUROPEAN PATENT SPECIFICATION

(11) **EP 4 053 272 B1**
(45) Date of publication and mention of the grant of the patent: **01.10.2025**
(21) Application number: 20894823.2
(22) Date of filing: 25.11.2020
(51) Int. Cl.: C12N 9/52, A23K 10/14, A23K 10/16, A23K 20/189, A23K 10/18, C12R 1/125

(54) **NOVEL SERINE PROTEASE VARIANT**
NEUARTIGE SERINPROTEASEVARIANTE
NOUVEAU VARIANT DE SÉRINE PROTÉASE

(30) Priority: 29.11.2019 KR 20190157400
(43) Date of publication of application: 07.09.2022
(73) Proprietor: CJ Cheiljedang Corporation, Seoul 04560 (KR)
(72) Inventor: LEE, Joo Hee, Seoul 04560 (KR); SUNG, Jinwoo, Seoul 04560 (KR); SEOK, Jong-cheol, Seoul 04560 (KR); LEE, Ji Hyun, Seoul 04560 (KR); SIM, Se Hoon, Seoul 04560 (KR); LEE, Imsang, Seoul 04560 (KR)
(74) Representative: Regimbeau
(86) International application number: PCT/KR2020/016775
(87) International publication number: WO 2021/107588

(56) References cited:
- EP-A2- 2 589 651
- WO-A1-2014/209789
- WO-A1-2018/015304
- WO-A1-2018/118815
- US-A1- 2003 073 222
- US-A1- 2018 340 191
- US-A1- 2019 032 102
- US-B2- 9 441 215
- TAEWAN KIM ET AL: "Expression and characterization of a thermostable serine protease (TfpA) from Thermomonospora fusca YX in Pichia pastoris", APPLIED MICROBIOLOGY AND BIOTECHNOLOGY, SPRINGER, BERLIN, DE, vol. 68, no. 3, 9 April 2005 (2005-04-09), pages 355 - 359, XP019331923, ISSN: 1432-0614, DOI: 10.1007/S00253-005-1911-8
- KELCH ET AL: "Mesophile versus Thermophile: Insights Into the Structural Mechanisms of Kinetic Stability", JOURNAL OF MOLECULAR BIOLOGY, ACADEMIC PRESS, UNITED KINGDOM, vol. 370, no. 4, 8 June 2007 (2007-06-08), pages 784 - 795, XP022107145, ISSN: 0022-2836, DOI: 10.1016/J.JMB.2007.04.078

## Description

### TECHNICAL FIELD

The present disclosure relates to a novel serine protease variant.

### BACKGROUND ART

Proteases are involved in various functions such as digestion, absorption, and defense in living organisms and are classified into serine proteases, cysteine proteases, aspartic proteases, and metalloproteases according to the structures of active sites. Among them, serine proteases (or serine endopeptidases) are enzymes characterized by commonly having an active serine residue in their active sites that cleave peptide bonds in proteins, in which serine serves as a nucleophilic amino acid at a protease's active site (Hedstrom, 2002. Chem Rev 102: 4501-4524).

Serine proteases have been used in a wide variety of applications. In addition to therapeutic applications to treat human diseases such as lysis of blood clots, serine proteases are used not only as components of laundry detergents and contact lens cleaners, but also used in modification of milk proteins, silk degumming, soaking of leather, unhairing, synthesis of oligopeptides, recovery of silver from lung X-ray films, production and improvement of feeds and foods (Korean Patent Publication No. 10-2005-0068750), etc. However, there is a need to develop a serine protease having improved thermal stability, increased activity, etc. to obtain higher industrial cost-effectiveness and efficiency.

Under such circumstances, the present inventors have made intensive efforts, and as a result, they have developed a novel variant of a serine protease derived from *Thermobifida fusca* and have found that the activity of the novel serine protease variant is far greater than those of conventional serine proteases, thereby completing the present disclosure.

### DESCRIPTION OF EMBODIMENTS

### TECHNICAL PROBLEM

An object of the present disclosure is to provide a serine protease variant.

Another object of the present disclosure is to provide a polynucleotide encoding the serine protease variant and a vector including the same.

Still another object of the present disclosure is to provide a microorganism expressing the serine protease variant.

Still another object of the present disclosure is to provide a feed composition including at least one between the serine protease variant and the microorganism expressing the same.

### TECHNICAL SOLUTION

The present disclosure will be described in detail.

The present invention corresponds to those claimed in the annexed set of claims.

An aspect of the present disclosure provides a serine protease variant comprising an amino acid sequence having a sequence identity of at least 80% and less than 100% with the amino acid sequence of SEQ ID NO: 31, wherein the amino acid corresponding to the 12^{th} position of the amino acid sequence of SEQ ID NO: 31 is substituted with a different amino acid and wherein said serine protease variant has an enzyme activity exceeding 100% compared to the protein having the serine protease activity before mutation.

As used herein, the term "serine protease" refers to an enzyme belonging to a sub-group of a protease and having a proteolytic activity. Specifically, the serine protease may be an enzyme which degrades proteins by hydrolyzing peptide bonds and basically has an active serine residue at active sites thereof, and more specifically, an enzyme which has a spatial arrangement of amino acid residues of histidine, aspartate, and serine, which can be referred to as a catalytic triad.

The serine protease disclosed herein may be derived from a microorganism of the genus *Thermobifida.* Particularly, in the present disclosure, a wild-type of the serine protease may be a serine protease derived from *Thermobifida fusca,* specifically, a polypeptide having an amino acid sequence derived from SEQ ID NO: 40 or an amino acid sequence derived from SEQ ID NO: 2, and more specifically, a polypeptide including an amino acid sequence as set forth in SEQ ID NO: 31,. The amino acid sequences of SEQ ID NOS: 2, 31, and 40 may be obtained from known database such as GenBank database of the National Center for Biotechnology Information (NCBI).

The serine protease of the present disclosure may have any sequence having the same activity as that of the amino acid sequence described above. The serine protease may have an amino acid sequence having at least 80% homology or identity with the amino acid sequence of SEQ ID NO: 31. Specifically, the serine protease may include amino acids having at least 80%, 85%, 90%, 91%, 92%, 93%, 94%, 95%, 96%, 97%, 98%, or 99% homology or identity with SEQ ID NO: 31. Additionally, it is obvious that any protein having an amino acid sequence including deletion, modification, substitution, or addition in part of the sequence is within the scope of the present disclosure as long as the amino acid sequence has the homologies or identities described above and an effect equivalent to that of the serine protease.

In other words, although the expressions 'protein or polypeptide having an amino acid sequence as set forth in a predetermined SEQ ID NO:' and 'protein or polypeptide including an amino acid sequence as set forth in a predetermined SEQ ID NO:' are used in the present disclosure, it is obvious that any protein having the amino acid sequence including deletion, modification, substitution, or addition in part of the sequence may also be used in the present disclosure, as long as the protein has an activity identical or equivalent to the polypeptide consisting of the corresponding amino acid sequence. For example, it is obvious that the 'polypeptide including an amino acid sequence of SEQ ID NO: 31' belongs to the 'polypeptide consisting of the amino acid sequence of SEQ ID NO: 31' as long as the former has an activity identical or equivalent to the latter.

As used herein, the term 'homology' or 'identity' refers to a degree of relevance between two given amino acid sequences or nucleotide sequences and may be shown as a percentage. The terms homology and identity may be used interchangeably.

Sequence homology or identity of conserved polynucleotides or polypeptides may be determined by standard alignment algorithm and default gap penalties established by a program being used may be used together. Substantially, homologous or identical sequences may hybridize with each other along the entire length or at least about 50%, 60%, 70%, 80% or 90% of the entire sequence under moderate or highly stringent conditions. In hybridized polynucleotides, polynucleotides including degenerated codons instead of codons are also considered.

The sequence homology, similarity, or identity between any two given polynucleotides or polypeptides may be determined using a known computer algorism such as "FASTA" program using default parameters as introduced by, for example, Pearson et al (1988) [Proc. Natl. Acad. Sci. USA 85: 2444]. Alternatively, Needleman-Wunsch algorithm (1970, J. Mol. Biol. 48: 443-453) performed in a Needleman program of the European Molecular Biology Open Software Suite (EMBOSS) package (Rice et al., 2000, Trends Genet. 16: 276-277) (version 5.0.0 or later) may be used to determine the same (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity may be determined using BLAST from The National Center for Biotechnology Information database, or ClustalW.

The homology, similarity, or identity between polynucleotides or polypeptides may be determined by comparing sequence information using a GAP computer program, such as a program introduced by Needleman et al. (1970), J Mol Biol. 48 : 443 as disclosed in Smith and Waterman, Adv. Appl. Math (1981) 2:482. In brief, the GAP program defines similarity as the number of aligned symbols (i.e., nucleotides or amino acids) which are similar, divided by the total number of symbols in a shorter of two sequences. Default parameters for the GAP program may include: (1) a binary comparison matrix (containing a value of 1 for identity and 0 for non-identity) and a weighted comparison matrix of Gribskov et al. (1986) Nucl. Acids Res. 14: 6745 disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

In addition, the sequence homology, similarity, or identity between any two given polynucleotides or polypeptides may be confirmed by comparing sequences thereof by southern hybridization under defined stringent conditions, and the defined proper hybridization conditions are within the scope of the technology and may be determined by a method well known to one of ordinary skill in the art.

The serine protease variant provided in the present disclosure may refer to a variant in which an amino acid at a specific position is substituted so as to have an enzyme activity exceeding 100% compared to the protein before mutation, among the above-described proteins having the serine protease activity.

As used herein, the term "variant" refers to a polypeptide obtained by conservative substitution and/or modification of at least one amino acid different from that of the recited sequence while retaining functions or properties of the protein. A variant has an amino acid sequence different from the identified sequence due to substitution, deletion, or addition of several amino acids. Such a variant may generally be identified by modifying one of the above polypeptide sequences and evaluating properties of the modified polypeptide. That is, the ability of a variant may be enhanced, unchanged, or diminished relative to a native protein.

In addition, some variants may include variants in which at least one portion such as an N-terminal leader sequence or transmembrane domain is removed. Other variants may include variants in which a portion is removed from or added to the N- and/or C-terminus of a mature protein.

The term "variant" may also be used interchangeably with other terms such as modified/mutated protein, modification, modified polypeptide, mutant, mutein, and divergent, and any terms used to indicate variation may also be used without limitation.

The variant may have an enhanced activity compared to natural wild-type or non-modified proteins, without being limited thereto.

As used herein, the term "conservative substitution" refers to substitution of one amino acid with a different amino acid having similar structural and/or chemical properties. For example, the variant have at least one conservative substitution while retaining at least one biological activity. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamate and aspartate; aromatic amino acids include phenylalanine, tryptophan, and tyrosine; and hydrophobic amino acids include alanine, valine, isoleucine, leucine, methionine, phenylalanine, tyrosine, and tryptophan. Additionally, amino acids may be classified into electrically charged amino acids with side chains and uncharged amino acids with side chains. The electrically charged amino acids with side chains include aspartic acid, glutamate, lysine, arginine, and histidine, and the uncharged amino acids with side chains are classified into nonpolar amino acids or polar amino acids. The nonpolar amino acids include glycine, alanine, valine, leucine, isoleucine, methionine, phenylalanine, tryptophan, and proline, and the polar amino acids include serine, threonine, cysteine, tyrosine, asparagine, and glutamine. In general, conservative substitution has little or no influence on the activity of a produced polypeptide.

The variant may also include deletion or addition of amino acids having a minimal influence on properties and a secondary structure of the polypeptide. For example, the polypeptide may be conjugated to a signal (or leader) sequence of the N-terminal of a protein involved in transfer of the protein co-translationally or post-translationally. The polypeptide may also be conjugated to a different sequence or linker to identify, purify, or synthesize the polypeptide.

As used herein, the term "serine protease variant" refers to a polypeptide including substitution of at least one amino acid in an amino acid sequence of a polypeptide having the serine protease activity. The serine protease variant according to the present disclosure includes variants in which an amino acid corresponding to position 12 from the N-terminus of the amino acid sequence of SEQ ID NO: 31 is substituted with a different amino acid. Specifically, the variant may be a protein in which the amino acids at positions 12 and 116 from the N-terminus of the amino acid sequence of SEQ ID NO: 31 are each substituted with a different amino acid. The term 'different amino acid' means an amino acid which is different from the amino acid before substitution, and any amino acid different from that before substitution may be used without limitation.

Specifically, the serine protease variant according to the present disclosure may be a variant in which the phenylalanine at position 12 of the amino acid sequence of SEQ ID NO: 31 is substituted with glycine, alanine, arginine, aspartate, cysteine, glutamate, asparagine, glutamine, histidine, proline, serine, tyrosine, isoleucine, leucine, lysine, tryptophan, valine, methionine, or threonine; and/or the asparagine at position 116 is substituted with glycine, alanine, arginine, aspartate, cysteine, glutamate, glutamine, histidine, proline, serine, tyrosine, isoleucine, leucine, lysine, phenylalanine, tryptophan, valine, methionine, or threonine, without being limited thereto.

Specifically, the variant may be a protein in which an amino acid corresponding to position 12 of the amino acid sequence of SEQ ID NO: 31 is substituted with tyrosine (Y), serine (S), alanine (A), or arginine (R); an amino acid corresponding to position 116 of the amino acid sequence is substituted with aspartate (D), serine (S), threonine (T), or glycine (G); or amino acids at positions 12 and 116 of the amino acid sequence of SEQ ID NO: 31 are each substituted with tyrosine (Y) and aspartate (D), tyrosine (Y) and serine (S), serine (S) and aspartate (D), serine (S) and threonine (T), or alanine (A) and glycine (G), without being limited thereto. It is obvious that the variant, in which the amino acid at position 12 or 12^{th} and 116 of the amino acid sequence of SEQ ID NO: 31 are each substituted with a different amino acid, includes variants in which the amino acids corresponding to each of the positions are each substituted with a different amino acid.

Additionally, the variant also includes variants in which the amino acids at positions corresponding to the 12^{th} and 116^{th} amino acids from the N-terminus of the SEQ ID NO: 31 are each substituted with a different amino acid in the amino acid sequence as set forth in SEQ ID NO: 31 or amino acid sequences having at least 80%, 85%, 90%, 91%, 92%, 93%, 94%, 95%, 96%, 97%, 98%, or 99% homology or identity with the amino acid sequence of SEQ ID NO: 31.

Specifically, among the above-described variants, the variants in which the amino acids at positions corresponding to the 12 ^{th} or 12^{th} and 116 ^{th} amino acids of the amino acid sequence of SEQ ID NO: 31 are each substituted with a different amino acid may consist of any one amino acid sequence selected from the amino acid sequences of SEQ ID NOS: 32 to 39, without being limited thereto. These variants may be those having a serine protease activity in which the amino acids at positions corresponding to the 12^{th} or 12^{th} and 116^{th} positions of the amino acid sequence of SEQ ID NO: 31 are each substituted with a different amino acid, and have a homology to the amino acid sequence of SEQ ID NO: 31 of at least 80%, 90%, 95%, 96%, 97%, 98%, or 99% and less than 100%.

The serine protease variant of the present disclosure may have an enhanced activity compared to a polypeptide before modification, a natural wild-type polypeptide, or a non-modified polypeptide, without being limited thereto. In addition, it is obvious that any protein having an amino acid sequence having deletion, modification, substitution, and addition in part of the amino acid sequence is within the scope of the present disclosure as long as the protein has the above-described homologies and an effect equivalent to that of the serine protease.

In addition, it is obvious that any variant having addition of a sequence in the forward or reverse direction of the amino acid sequence of the corresponding SEQ ID NO or a naturally occurring mutation, or a silent mutation , in addition to the mutations in the 12^{th} or 12^{th} and116^{th} amino acids providing a specific activity or mutations at positions corresponding thereto, are not excluded from the scope of the present disclosure, as long as the variant has an activity identical or equivalent to the variant according to the present disclosure.

Meanwhile, a mature region of NCBI Reference Sequence WP_016188200.1 (SEQ ID NO: 40) corresponds to the amino acid sequence of SEQ ID NO: 31 of the present disclosure, and the sequence excluding the signal peptide from SEQ ID NO: 40 corresponds to SEQ ID NO: 2 of the present disclosure.

It is obvious as described above that the serine protease variant of the present disclosure may include deletion or addition of amino acids having a minimum influence on the properties and the secondary structure of the serine protease, in which the amino acids at positions corresponding to the 12 ^{th} or 12^{th} and 116 ^{th} amino acids of SEQ ID NO: 31 are each substituted with a different amino acid. In addition, it would be obvious to those skilled in the art, through a sequence alignment well known in the art, that the 12^{th} and 116^{th} positions from the N-terminus of SEQ ID NO: 31 according to the present disclosure correspond to the 193^{rd} and 297^{th} positions of SEQ ID NO: 40 and the 163^{rd} and 267^{th} positions of SEQ ID NO: 2, and that SEQ ID NO: 31 is included in both SEQ ID NO: 40 and SEQ ID NO: 2.

Thus, with respect to the amino acid sequences of SEQ ID NOS: 2 and 40 each including the amino acid sequence of SEQ ID NO: 31, the serine protease variant of the present disclosure includes variants in which the amino acids at positions corresponding to the 12^{th} and 116^{th} amino acids of SEQ ID NO: 31 (the 163^{rd} and/or 267^{th} amino acids in SEQ ID NO: 2 and the 193^{rd} and/or 297^{th} amino acids in SEQ ID NO: 40) are each substituted. Additionally, the descriptions given above with reference to SEQ ID NO: 31 and the 12^{th} and 116^{th} amino acids thereof can also be applied to SEQ ID NO: 2 and the 163^{rd} and 267^{th} amino acids thereof and SEQ ID NO: 40 and the 193^{rd} and 297^{th} amino acids thereof.

According to an embodiment, the serine protease variant of the present disclosure may be a variant, which has an amino acid sequence where amino acids at positions corresponding to the 12^{th} or 12^{th} and 116^{th} positions of SEQ ID NO: 31 are each substituted with a different amino acid and has a homology to the amino acid sequence of SEQ ID NO: 2 of at least 80% and less than 100%. According to another embodiment, the serine protease variant of the present disclosure may be a variant in which the 163^{rd} and/or 267^{th} amino acids of SEQ ID NO: 2 are each substituted with a different amino acid, and has a homology to the amino acid sequence of SEQ ID NO: 2 of at least 80% and less than 100%, and has a homology to any one amino acid sequence selected from amino acid sequences of SEQ ID NOS: 3 to 10 of at least 80% , but is not limited thereto.

Another aspect of the present disclosure provides a polynucleotide encoding the serine protease variant of the present invention.

As used herein, the term "polynucleotide" refers to a polymer of nucleotides in which nucleotide monomers are connected with one another in a long chain shape by covalent bonds and generally means a DNA or RNA strand having a certain minimum length, and more specifically, a polynucleotide fragment encoding the variant.

The polynucleotide encoding the serine protease variant of the present disclosure may have any nucleotide sequence encoding the serine protease variant having an enhanced activity according to the present disclosure without limitation. In the present disclosure, a gene encoding a wild-type serine protease may be derived from a microorganism of the genus *Thermobifida,* and specifically *Thermobifida fusca,* without being limited thereto.

The polynucleotide of the present disclosure may include various modifications made in a coding region in the amino acid sequence of the polypeptide within a range not changing the amino acid sequence, due to codon degeneracy or in consideration of codons preferred by a living organism in which the polypeptide is to be expressed. Specifically, any nucleotide sequence encoding the variant in which the amino acids at positions corresponding to the 12 or 12 and 116 amino acids from the N-terminus of SEQ ID NO: 31 are each substituted with a different amino acid may be included therein.

For example, the polynucleotide of the present disclosure may be a polynucleotide sequence encoding the variant of the present disclosure, and specifically, encoding a polypeptide consisting of any one of the amino acid sequences as set forth in SEQ ID NOS: 32 to 39 or a polypeptide having a homology therewith, without being limited thereto. More specifically, the polynucleotide may be one consisting of a polynucleotide sequence as set forth in any one SEQ ID NO selected from SEQ ID NOS: 41 to 48, without being limited thereto. In addition, as described above, since the serine protease variant of the present disclosure includes those variants in which the amino acids corresponding to the 12^{th} or 12^{th} and 116^{th} amino acids of SEQ ID NO: 31 (the 163^{rd} and 267^{th} amino acids in SEQ ID NO: 2 and the 193^{rd} and/or 297^{th} amino acids in SEQ ID NO: 40) are each substituted, wherein the amino acid sequences of SEQ ID NOS: 2 and 40 include the amino acid sequence of SEQ ID NO: 31, the polynucleotide sequence encoding the serine protease variant is also within the scope of the present disclosure. For example, the polynucleotide sequence encoding the serine protease variant may be one which encodes an amino acid sequence as set forth in any one SEQ ID NO selected from SEQ ID NOS: 3 to 10, and specifically, may be one consisting of a polynucleotide sequence as set forth in any one SEQ ID NO selected from SEQ ID NOS: 23 to 30, without being limited thereto.

Additionally, any sequence, which encodes a protein having an activity of the variant in which the amino acids at positions corresponding to the 12^{th} or 12^{th} and 116 ^{th} amino acids from the N-terminus of SEQ ID NO: 31 are each substituted with a different amino acid by hybridizing with a probe that can be prepared from known gene sequences (*e.g*., sequences complementary to all or part of the nucleotide sequence) under stringent conditions, can be included without limitation.

The term "stringent conditions" refers to conditions which allow specific hybridization between polynucleotides. Such conditions are disclosed in detail in literature. For example, the stringent conditions may include performing hybridization between genes having a high homology, at least 40%, specifically at least 90%, more specifically at least 95%, even more specifically at least 97%, and most specifically at least 99% homology, while not performing hybridization between genes having a homology lower than the above homologies, or performing washing once, specifically twice or three times, under conventional washing conditions for southern hybridization of 60°C, 1×SSC, and 0.1% SDS, specifically at a salt concentration and a temperature of 60°C, 0.1×SSC, 0.1% SDS, and more specifically 68°C, 0.1×SSC, and 0.1% SDS. However, stringent conditions are not limited thereto, but may be appropriately adjusted by those skilled in the art according to the purpose thereof.

Hybridization requires that two nucleotides have complementary sequences, although bases may mismatch depending on the stringency of hybridization. The term "complementary" is used to describe the relationship between nucleotide bases which are capable of hybridizing with each other. For example, in the case of DNA, adenosine is complementary to thymine and cytosine is complementary to guanine. Thus, the present disclosure may include not only a substantially similar polynucleotide sequence but also an isolated polynucleotide fragment thereof complementary to the entire sequence.

Specifically, the polynucleotide having homology may be detected using the above-described hybridization conditions including a hybridization process at a Tm value of 55°C. Additionally, the Tm value may be 60°C, 63°C, or 65°C, but is not limited to and may be appropriately adjusted by those skilled in the art according to the purpose thereof.

An appropriate degree of stringency for hybridization of polynucleotides may depend on lengths of the polynucleotides and a degree of complementarity and parameters thereof are well known in the art.

Still another aspect of the present disclosure provides a vector including a polynucleotide encoding the serine protease variant of the present disclosure.

As used herein, the term "vector" refers to a DNA construct containing a nucleotide sequence of a target protein-encoding polynucleotide operably linked to a suitable control sequence so as to be able to express the target protein in a suitable host cell. The control sequence may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function regardless of the host genome, or may integrate into genome thereof.

As used herein, the term "operably linked" means that a polynucleotide sequence encoding the target protein of the present disclosure is functionally linked to a promoter sequence which initiates and mediates transcription of the polynucleotide. An operable linkage may be prepared by a genetic recombination technique known in the art, and site-specific DNA cleavage and ligation may be prepared using a restriction enzyme, a ligase, etc. known in the art, without being limited thereto.

The vector used in the present disclosure is not particularly limited, but any vector known in the art may be used. Examples of conventional vectors may include a plasmid, cosmid, virus and bacteriophage in a natural or recombinant state. For example, pWE15, M13, MBL3, MBL4, IXII, ASHII, APII, t10, t11, Charon4A, and Charon21A may be used as a phage vector or cosmid vector, and those based on pBR, pUC, pBluescriptII, pGEM, pTZ, pCL, pET, pUB110, etc. may be used as a plasmid vector. Specifically, vectors pDZ, pACYC177, pACYC184, pCL, pECCG117, pUC19, pBR322, pMW118, pCC1BAC, and pSM704, etc. may be used. The vector that can be used in the present disclosure is not particularly limited, but any known expression vector may be used.

In an embodiment, the polynucleotide encoding the target variant in a chromosome may be substituted with a mutated polynucleotide using a vector for chromosomal insertion into cells. The insertion of the polynucleotide into the chromosome may be performed by any method known in the art, for example, homologous recombination, without being limited thereto. The vector may further include a selection marker to confirm chromosomal insertion. The selection marker is used to select cells transformed with the vector, that is, to confirm whether a target nucleic acid molecule is inserted, and examples of the selection marker may include markers providing selectable phenotypes, such as drug resistance, auxotrophy, resistance to cytotoxic agents, or expression of surface mutant polypeptides. In an environment where a selective agent is treated, only cells expressing the selection marker can survive or show different phenotypes, and thus the transformed cells can be selected.

Still another aspect of the present disclosure provides a microorganism expressing the serine protease variant of the present disclosure.

As used herein, the term "to be expressed/being expressed/expressing" a protein means a state in which a target protein is introduced into a microorganism or expressed in a microorganism. In view of the objects of the present disclosure, the "target protein" may be the above-described serine protease variant.

Specifically, the term "introduction of a protein" refers to exhibiting an activity of a particular protein in a microorganism which originally does not possess the protein or exhibiting an enhanced activity of the protein compared to the endogenous activity of the protein or the activity before modification. For example, the introduction of a protein may refer to introduction of a polynucleotide encoding a particular protein into the chromosome of a microorganism or introduction of a vector including the polynucleotide encoding the particular protein into a microorganism, thereby exhibiting the activity of the protein.

The microorganism expressing the serine protease variant may include at least one among the serine protease variant of the present disclosure, a polynucleotide encoding the variant, and a vector including the polynucleotide.

Specifically, the microorganism including at least one among the serine protease variant, the polynucleotide encoding the variant, and the vector including the polynucleotide may be a microorganism which is prepared by transformation with the vector including the polynucleotide encoding the variant, without being limited thereto.

The microorganism may be a recombinant microorganism, and the recombination may be achieved by genetic modification such as transformation.

As used herein, the term "transformation" refers to a process of introducing a vector including a polynucleotide encoding a target protein into a host cell in such a way that the protein encoded by the polynucleotide is expressed in the host cell. Regardless of whether the transformed polynucleotide is in a form inserted into the chromosome of a host cell or in a form located outside the chromosome, the transformed polynucleotides in both forms are within the scope of the present disclosure, as long as the protein is expressed in the host cell. In addition, the polynucleotide includes DNA and RNA that encode the target protein. The polynucleotide may be introduced into a host cell in any form as long as the polynucleotide is introduced into the host cell and is expressed therein. For example, the polynucleotide may be introduced into a host cell in the form of an expression cassette, which is a gene construct including all of the essential elements required for self-replication. The expression cassette may generally include a promoter operably linked to the polynucleotide, a transcription termination signal, a ribosome binding site, and a translation termination signal. The expression cassette may be in the form of a self-replicable expression vector. Additionally, the polynucleotide may be introduced into a host cell in its original form and operably linked to a sequence required for the expression in the host cell, without being limited thereto. Methods for the transformation include any method used to introduce a polynucleotide into cells and may be performed by suitable standard techniques known in the art. For example, the transformation methods include electroporation, calcium phosphate (Ca(H₂PO₄)₂, CaHPO₄, or Ca₃(PO₄)₂) precipitation, calcium chloride (CaCl₂) precipitation, microinjection, a polyethylene glycol (PEG) method, a DEAE-dextran method, a cationic liposome method, natural competence (e.g., see Perry and Kuramitsu, 1981, Infect. Immun. 32: 1295-1297]), and a lithium acetate-DMSO method, without being limited thereto.

The recombinant microorganism may be a microorganism in which the activity of the serine protease of the present disclosure is enhanced.

The "enhancement of activity" may mean that the activity of a particular protein of a microorganism is enhanced compared to the endogenous activity or the activity before modification. The term "endogenous activity" may refer to an activity of a particular protein possessed by a parent strain of a microorganism before transformation when the microorganism is transformed by genetic mutation caused by a natural or artificial factor.

Specifically, the enhancement of the activity of the protein variant in the present disclosure may be achieved by at least one of the methods including a method of increasing the intracellular copy number of a gene encoding the protein variant, a method of introducing mutation into an expression control sequence of the gene encoding the protein variant, a method of replacing the expression control sequence of the gene encoding the protein variant with a sequence having a stronger activity, a method of replacing a chromosomal gene encoding a wild-type protein having the serine protease activity with a gene encoding the protein variant, and a method of further introducing mutation into the gene encoding the protein variant in order to enhance the activity of the protein variant, without being limited thereto.

Next, the modification of the expression control sequence to increase the expression of a polynucleotide may be performed by inducing a mutation in the nucleic acid sequence by deletion, insertion, non-conservative substitution, conservative substitution, or a combination thereof to further enhance the activity of the expression control sequence, or by replacing the nucleic acid sequence with a nucleic acid sequence having a stronger activity, without being limited thereto. The expression control sequence may include a promoter, an operator sequence, a ribosome-binding site encoding sequence, sequences for regulating transcription and translation, etc. without being limited thereto.

A strong promoter, instead of the endogenous promoter, may be linked upstream of the polynucleotide expression unit, but the present disclosure is not limited thereto. Examples of the strong promoter may include CJ1 to CJ7 promoters (Korean Patent No. 10-0620092), a lac promoter, a trp promoter, a trc promoter, a tac promoter, a lambda phage PR promoter, a P_{L} promoter, a tet promoter, a gapA promoter, an SPL7 promoter, an SPL13 (sm3) promoter (Korean Patent No. 10-1783170), an O2 promoter (Korean Patent No. 10-1632642), a tkt promoter, a yccA promoter, etc. without being limited thereto.

In addition, the modification of the polynucleotide sequence on the chromosome may be performed by inducing a mutation in the expression control sequence by deletion, insertion, non-conservative substitution, conservative substitution, or a combination thereof to further enhance the activity of the polynucleotide sequence, or by replacing the sequence with a polynucleotide sequence modified to have a stronger activity, without being limited thereto.

Generally, the introduction and enhancement of a protein activity may increase the activity or concentration of the protein by 1%, 10%, 25%, 50%, 75%, 100%, 150%, 200%, 300%, 400%, or 500%, to a maximum of 1,000% or 2,000%, from the activity or concentration in a wild-type or non-modified microorganism strain, without being limited thereto.

The host cell or microorganism according to the present disclosure may be any microorganism expressing the serine protease variant by including the polynucleotide of the present disclosure or the vector of the present disclosure. Specifically, examples of the host cell or microorganism may include strains of microorganisms belonging to the genus *Escherichia,* the genus *Serratia,* the genus *Erwinia,* the genus *Enterobacteria,* the genus *Providencia,* the genus *Salmonella,* the genus *Streptomyces,* the genus *Pseudomonas,* the genus *Brevibacterium,* the genus *Corynebacterium,* or the genus *Bacillus,* etc., specifically, may be strains of *Bacillus subtilis, Bacillus licheniformis, Bacillus amyloliquefaciens, Bacillus velezensis, Escherichia coli, Corynebacterum glutamicum, or Aspergillus oryzae,* and more specifically, may be *Bacillus subtilis,* without being limited thereto.

Still another aspect of the present disclosure provides a feed composition including at least one between the serine protease variant of the present disclosure and a microorganism expressing the same.

The serine protease variant to be included in the feed composition of the present disclosure variant may be included in the feed composition in such a manner that the microorganism expressing the serine protease variant is included therein or may be in a form isolated from the microorganism expressing the same and purified, but the present disclosure is not limited thereto.

As used herein, the term "feed composition" refers to any preparation of any natural or artificial diet, meal, etc. or components of the meal for animals to eat, intake, and digest or being suitable therefor, and the feed may be prepared in various forms known in the art.

The feed composition may be a feed additive.

Types of the feed are not particularly limited, and feed commonly used in the art may be used. Non-limiting examples of the feed may include: vegetable feeds such as grains, roots/fruits, food processing by-products, algae, fibers, pharmaceutical by-products, oils and fats, starches, gourds, and grain by-products; and animal feeds such as proteins, inorganic materials, oils and fats, minerals, single-cell proteins, animal planktons, or foods. These feeds may be used alone or in combination of at least two thereof.

The feed composition of the present disclosure may further include at least one selected from: an organic acid such as citric acid, fumaric acid, adipic acid, lactic acid, and malic acid; a phosphate such as sodium phosphate, potassium phosphate, acid pyrophosphate, and polyphosphate (polymerized phosphate); and a natural anti-oxidant such as polyphenol, catechin, alpha-tocopherol, a rosemary extract, vitamin C, a green tea extract, a licorice root extract, chitosan, tannic acid, and phytic acid.

The feed composition of the present disclosure may further include at least one selected from: an auxiliary substance such as amino acids, minerals, vitamins, antibiotics, antibacterial substances, antioxidants, antifungal enzymes, and microbial preparations in different probiotic forms; grains, (e.g., pulverized or crushed wheat, oat, barley, corn, and rice); vegetable protein feeds including rape, beans, and sunflowers as a main ingredient; animal protein feeds such as powders of blood, meat, bone, and fish; sugar and dairy products (e.g., dry components formed of various types of dry milk and whey powder; lipids (e.g., active ingredients such as any animal fats and vegetable oils liquefied by heating); and additives such as nutritional supplements, digestion and absorption promoters, growth promoters, and prophylactic agents.

The feed composition of the present disclosure may be in the form of a dry or liquid preparation and may further include an excipient for feed. The excipient for feed may be, for example, zeolite, corn flour, rice bran, etc., without being limited thereto.

The feed composition of the present disclosure may further include an enzyme preparation in addition to the serine protease variant. For example, the feed composition may further include at least one selected from a lipid-degrading enzyme such as lipase, phytase which degrades phytic acid into phosphate and inositol phosphate, amylase which is an enzyme catalyzing the hydrolysis of α-1,4-glycoside bonds included in starch, glycogen, etc., phosphatase which is an enzyme catalyzing the hydrolysis of organophosphate ester, maltase which catalyzes maltose into two glucose molecules, and a converting enzyme which catalyzes the hydrolysis of sucrose into a glucose-fructose mixture. However, the present disclosure is not limited thereto.

The feed composition of the present disclosure may be administered to animals alone or in combination with other feed additives included in an edible carrier. In addition, the feed composition may be easily administered as a feed additive or top dressing, directly into a livestock feed or separately from the feed, or in a separate oral formulation or in combination with other ingredients. In addition, a daily dosage may be employed via once-daily dose or multiple-divided daily dose as commonly known in the art to which the present disclosure pertains.

Examples of animals to which the feed composition of the present disclosure is applied may include livestock such as beef cattle, dairy cattle, calves, pigs, piglets, sheep, goats, horses, rabbits, dogs, and cats; and poultry such as chicks, hens, domestic chickens, roosters, ducks, geese, turkeys, quails, and small birds, without being limited thereto.

The amount of the serine protease variant included in the feed composition of the present disclosure is not particularly limited and may be appropriately adjusted according to the purpose thereof. In an embodiment, the serine protease variant may be included in an appropriate amount for degrading a protein source material while surviving in the digestive tract of livestock for a long period of time as commonly known in the art to which the present disclosure pertains, but the present disclosure is not limited thereto.

Still another aspect of the present disclosure provides a food composition including at least one between the serine protease variant of the present disclosure and a microorganism expressing the same. The serine protease variant may be used in a liquid or solid food composition. In addition, the food may be powders, pills, beverage, tea, or an additive for general foods.

In an embodiment, the food may be a group of foods requiring a protease, such as dairy products, health functional foods for improving bowel movement and weight loss, and health functional foods for preventing hypertension.

In another embodiment, the serine protease variant may be included in various food compositions as a food solubilizer, food softening agent, and meat modifier. In various other embodiments, the serine protease variant may be added to baking mixes in a step for gluten network breakdown. Alternatively, the serine protease variant may be used to catalyze hydrolysis of food proteins (e.g., milk proteins). Alternatively, the serine protease variant may be included in various food compositions for the purpose of rendering, preparing flavoring agents, reducing bitterness, changing emulsifying properties, producing bioactive peptides, reducing allergy-causing antigens in proteins. However, this is only an illustrative embodiment and the uses of the serine protease variant are not limited thereto.

The amount of the serine protease variant included in the food composition of the present disclosure may be appropriately adjusted by those of ordinary skill in the art according to the purpose thereof.

It is also disclosed herein but not claimed a detergent composition including at least one between the serine protease variant of the present disclosure and a microorganism expressing the same.

The detergent composition of the present disclosure may be in the form of a first and second aqueous detergent composition, a non-aqueous liquid detergent composition, a cast solid, granules, particles, compressed tablets, gel, paste, or slurry. The detergent composition may be used to remove stubborn food stains, food residue films, and other small amounts of food compositions.

The detergent composition according to the present disclosure may be provided in the form of a detergent composition for cleaning hard surfaces, a detergent composition for cleaning fabrics, a detergent composition for dishwashing, a detergent composition for oral cleaning, a detergent for cleaning dentures, or a contact lens cleaning solution. However, the present disclosure is not limited thereto.

It is also described herein but not claimeda pharmaceutical composition including at least one between the serine protease variant of the present disclosure and a microorganism expressing the same.

The pharmaceutical composition of the present disclosure may be used as a pharmaceutical composition for digestive enzymes to improve digestive diseases, digestive disorders, and abnormalities after digestive surgery, a thrombolytic or antithrombotic composition directly applied to blood clots to dissolve fibrin, an anti-inflammatory drug acting as an *in vivo* defense system to remove inflammatory substances or necrotic tissue, or an anti-inflammatory drug to alleviate edema after surgery or wounds.

The pharmaceutical composition may further include a pharmaceutically acceptable carrier, excipient, diluent, or accessory ingredient according to the methods or purposes of use. The carrier, excipient, or diluent may include at least one selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxy benzoate, propyl hydroxy benzoate, talc, magnesium stearate, mineral oils, dextrin, calcium carbonate, propylene glycol, liquid paraffin, and saline, without being limited thereto.

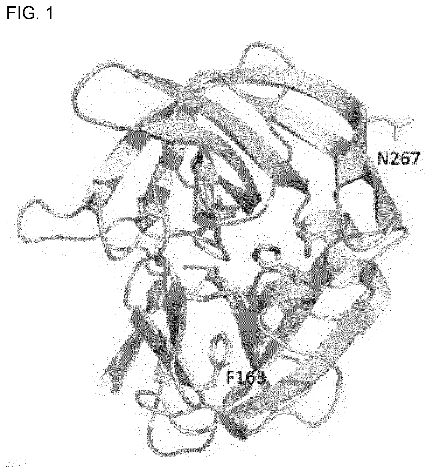

FIG. 1 is a ribbon diagram illustrating positions of residues into each of which a mutation is introduced in a tertiary structure of a *Thermobifida fusca*-derived serine protease variant.

### ADVANTAGEOUS EFFECTS OF DISCLOSURE

The serine protease variant of the present disclosure may be efficiently used in various industrial fields due to its enhanced activity compared to conventional serine proteases and an activity at high temperature and heat resistance.

### BRIEF DESCRIPTION OF DRAWINGS

The serine protease variant of the present disclosure may be efficiently used in various industrial fields due to its enhanced activity compared to conventional serine proteases and an activity at high temperature and heat resistance.

### BEST MODE

Hereinafter, the present disclosure will be described in more detail with reference to the following examples and experimental examples.

### Example 1. Selection of Serine Protease Variant Derived from Thermobifida fusca

### Example 1-1: Preparation of Thermobifida fusca-derived Serine Protease Library

Random mutation was introduced into a gene encoding an amino acid (SEQ ID NO: 31) corresponding to a mature region of a *Thermobifida* fusca-derived serine protease by error-prone PCR. The Error prone PCR was performed using a Diversify^{™} PCR Random Mutagenesis Kit (Clontech, Cat No. 630703), and the PCR conditions used are as described in Table 1 below. It was confirmed that the mutation was introduced at a frequency of 6.2 mutations/kb.

**Table 1**

| Composition | Amount | Remarks |
|---|---|---|
| Template DNA | 0.5 ng | SEQ ID NO: 1 |
| Primer_forward | 5 µM | SEQ ID NO: 11 |
| Primer_reverse | 5 µM | SEQ ID NO: 12 |
| 10X Titanium Taq buffer | 5 µL | |
| MnSO₄ | 640 µM | |
| dGTP | 40 µM | |
| 50X Diversify dNTP mix | 1 µL | |
| 50X dNTP mix | 1 µL | |
| Titanium Taq Polymerase | 1 µL | |
| Total | 50 µL | Adjust to 50 µL with DW |

| PCR Conditions | | |
|---|---|---|
| I. 94°C | 30 sec | Repeat 50 cycles of II and III |
| II. 94°C | 30 sec | |
| III. 68°C | 1 min | |
| IV. 68°C | 1 min | |
| V. 4°C | ∞ | |

PCR fragments obtained in the above process were each ligated to a vector amplified using primers shown in Table 2 below using an In-FusionR HD cloning kit (Clontech) and transformed into DH5α cells to obtain colonies. The plasmids in the obtained colonies were purified to obtain a library having a size of about 5 × 10⁴.

**Table 2**

| | |
|---|---|
| Template DNA (pBE-S-TAP) | SEQ ID NO: 1 |
| Primer_forward | SEQ ID NO: 13 |
| Primer_reverse | SEQ ID NO: 14 |

### Example 1-2: Screening of Thermobifida fusca-derived Serine Protease Library

A *Bacillus subtilis* LB700 strain, which easily releases proteins, was transformed with the protease library prepared in Example 1-1 and screened. The screening was performed by a two-stage process. In a first stage, the *Bacillus subtilis* strain transformed with the library was plated on a 2% skim milk plate, and the desired colonies were selected based on the halo size. The transformation of *Bacillus subtilis* was performed according to the Groningen method and compositions of the skim milk used in the screening are as shown in Table 3 below.

**Table 3**

| Composition | Amount | Remarks |
|---|---|---|
| M9 minimal salts | 11.28 g | BD, Cat. No. 248510 |
| Agar | 20 g | |
| Skim milk | 20 g | BD, Cat. No. 232100 |
| 50%Glucose | 20 g | |
| 1 M MgSO₄ | 2 mM | |
| 1 M CaCl₂ | 0.1 mM | |
| Kanamycin | 50 µg/mL | |

### (per 1L)

A second stage relates to a method of re-selecting the colonies, which were selected in the first stage, by azocasein color development. A Brain Heart Infusion (BHI, bd, Cat. No. 53286) liquid medium containing a kanamycin antibiotic was added to a 96 deep well plate, and the colonies selected in the first stage were inoculated thereinto, followed by incubation at 37°C for 20 to 24 hours. After the incubation, a supernatant including an enzyme was obtained by centrifugation, and the supernatant was mixed with an equal amount of 2% (w/v) azocasein, as a substrate, followed by a reaction at 37°C for 1 hour. The reaction was terminated by adding a 3-fold volume of 10% trichloro acetic acid (TCA) to the enzyme reaction solution and coagulated proteins were removed by centrifugation. The color development reaction was performed by mixing the resultant with an equal amount of NaOH, and then the absorbance was measured at 440 nm to compare the degree of color development. Through this process, colonies having an increase in absorbance by 150% or more compared to the wild-type serine protease were selected.

### Example 2. Preparation of Selected Variant and Evaluation of Activity

### Example 2-1: Preparation of Variant

As a result of analyzing the sequences of variants selected from screening, it was confirmed that the 12^{th} amino acid (phenylalanine, Phe) and the 116^{th} amino acid (asparagine, Asn) of SEQ ID NO: 31 were substituted with tyrosine (Tyr) and aspartate (Asp), respectively. In FIG. 1, the positions of the mutations are indicated. After the two selected variants (F12 and N116) were re-introduced into pBE-S-TAP plasmid in the form of single mutation by site directed mutagenesis, the activities of the strains with a double mutation and a single mutation, respectively, were compared with the activity of the wild-type strain. Primers used to prepare the variants are as shown in Table 4 below.

**Table 4**

| | |
|---|---|
| TAP_F12Y_F | SEQ ID NO: 15 |
| TAP_F12Y_R | SEQ ID NO: 16 |
| TAP_N116D_F | SEQ ID NO: 17 |
| TAP_N116D_R | SEQ ID NO: 18 |

### Example 2-2: Evaluation of Activity

After the *Bacillus subtilis* LB700 strain was transformed with the prepared plasmid, the activity evaluation of the transformant was performed using a N-SUCCINYL-ALA-ALA-PRO-PHE-P-NITROANILIDE (Sigma, cat#S7388, hereinafter, referred to as SUC-AAPF-pNA) peptide as a substrate. The transformed *Bacillus subtilis* strain was inoculated into the Brain Heart Infusion (BHI, bd, cat#53286) liquid medium containing a kanamycin antibiotic and cultured at 37°C for 20 to 24 hours, and a portion of the culture solution except for the cells was mixed with a 25 mM Tris-HCl (pH7.5) buffer and a 1 mM Suc-AAPF-pNA, followed by reaction at 37°C for 30 minutes. The absorbance of the reaction solution was measured at 410 nm. An extinction coefficient of para-nitroaniline produced by the enzyme known in a literature was 8,800M⁻¹ cm⁻¹ at 410 nm, and the unit of the enzyme was calculated based thereon (Barrett, A. J., Cathepsin G. Methods Enzymol., 80, Pt. C, 561-565, (1981)). The measured activities are shown in Table 5 below.

**Table 5**

| | Enzyme activity (unit/ml) |
|---|---|
| Wild-type | 16.3 |
| F12Y | 34.0 |
| F12YN116D | 63.8 |

As a result of the measurement, it was confirmed that the activities of the F12Y and F12YN116D variants were increased in conditions at pH 7.5 and 37°C by about 2.1- and 3.9-fold, respectively.

### Example 2-3: Evaluation of Thermal Stability

Since thermal stability is a very important property of the enzyme, an experiment described below was performed to confirm the influence of introducing a variant on thermal stability.

Specifically, the enzyme activities of the samples used in Example 2-2 for the activity evaluation were measured after placing the samples at room temperature, at 70°C, at 80°C, and at 90°Cfor 5 minutes, respectively. The measured activities are shown in Table 6 below.

**Table 6**

| | Enzyme activity (unit/mL) | | | |
|---|---|---|---|---|
| Heat treatment conditions | RT, 5 min | 70°C, 5min | 80°C, 5 min | 90°C, 5 min |
| Wild-type | 16.3 | 15.9 | 10.6 | 0.3 |
| F12Y | 34.0 | 34.7 | 22.8 | 0.1 |
| F12YN116D | 63.8 | 63.3 | 41.7 | 0.1 |

As a result of the measurement, it was confirmed that the F12Y and F12YN116D mutation exhibited about 2- and 4-fold higher enzyme activities than the wild-type strain even at 80°C, respectively. Since it was confirmed that a high activity of the serine protease variant of the present disclosure was maintained even at high temperature, the serine protease variant can be effectively used in the industry.

### Example 3. Preparation and Selection of Saturation Mutagenesis Library

### Example 3-1. Preparation of Saturation Mutagenesis Library of F12 and N116 Residues

In order to identify the effects of substitution of each of the residues of F12 and N116 (i.e., previously selected variants) with a residue other than tyrosine and aspartate on their activities, saturation mutagenesis libraries were prepared with respect to the two residues.

Two PCR fragments were obtained using the pBE-S-TAP plasmid as a template and the primer pairs of SEQ ID NOS: 11 and 12 and SEQ ID NOS: 13 and 14, respectively. The fragments were each ligated using the In-Fusion HD cloning kit and then transformed into DH5α cells, thereby obtaining colonies. The plasmids in the obtained colonies were purified to obtain a library having a size of about 4 × 10³.

**Table 7**

| | |
|---|---|
| Template DNA (pBE-S-TAP) | SEQ ID NO: 1 |
| Saturation mutagenesis_F_1 | SEQ ID NO: 19 |
| Saturation mutagenesis_R_1 | SEQ ID NO: 20 |
| Saturation mutagenesis_F_2 | SEQ ID NO: 21 |
| Saturation mutagenesis_R_2 | SEQ ID NO: 22 |

### Example 3-2: Screening of Saturation Mutagenesis Library and Activity Evaluation

The saturation mutagenesis libraries prepared in Example 3-1 were subjected to screening in the same manner as in Example 1-2. Variants having the same or increased activity compared to the F12YN116D variant were selected by screening, and activity evaluation was performed on these variants by sequence analysis using Suc-AAPF-pNA as a substrate.

**Table 8**

| | Enzyme activity (unit/mL) |
|---|---|
| Wild-type | 23.52 |
| F12**Y**N116**D** | 65 |
| F12**Y**N116**S** | 77.35 |
| F12**S**N116**D** | 61.75 |
| F12**S**N116**T** | 117.65 |
| F12**A**N116**G** | 94.9 |
| F12**A** | 94.25 |
| F12**R** | 58.5 |

As a result, it was found that the F12 and N116 variants increased their activities even when their residues were each substituted with a different amino acid (e.g., F12S, F12A, F12R, N116S, N116T, N116G, etc.) in addition to tyrosine and aspartate confirmed in Example 2.

From the above results, it was confirmed that the F12 and N116 residues are important residues for exhibiting the activity of the serine protease, and the enzyme activity of the serine protease can be increased by substituting each of these residues with a different amino acid. Therefore, the serine protease variant of the present disclosure with an increased enzyme activity can be effectively used in the industry.

## Claims

1. A serine protease variant comprising an amino acid sequence having a sequence identity of at least 80% and less than 100% with the amino acid sequence of SEQ ID NO: 31, wherein the amino acid corresponding to the 12^{th} position of the amino acid sequence of SEQ ID NO: 31 is substituted with a different amino acid and wherein said serine protease variant has an enzyme activity exceeding 100% compared to the protein having the serine protease activity before mutation.

2. The serine protease variant according to claim 1, wherein the amino acid corresponding to the 116^{th} position of the amino acid sequence of SEQ ID NO: 31 is further substituted with a different amino acid.

3. The serine protease variant according to claim 1, wherein the amino acid corresponding to the 12^{th} position is substituted with tyrosine (Y), alanine (A), serine (S), or arginine (R).

4. The serine protease variant according to claim 2, wherein the amino acid corresponding to the 116^{th} position is substituted with aspartate (D), serine (S), threonine (T), or glycine (G).

5. The serine protease variant according to claim 1 or 2, which has a sequence identity to SEQ ID NO: 2 of at least 80% and less than 100%.

6. The serine protease variant according to claim 1 or 2, wherein the serine protease variant comprises an amino acid sequence as set forth in any one selected from the group consisting of SEQ ID NOS: 3 to 10 and SEQ ID NOS: 32 to 39.

7. A polynucleotide encoding the serine protease variant of any one of claims 1 to 6.

8. A vector comprising the polynucleotide of claim 7.

9. A microorganism expressing the serine protease variant of any one of claims 1 to 6.

10. The microorganism according to claim 9, wherein the microorganism belongs to the genus *Bacillus.*

11. The microorganism according to claim 10, wherein the microorganism is *Bacillus subtilis.*

12. A feed composition comprising the serine protease variant of claim 1 or 2 and/or a microorganism expressing the same.

## Patentansprüche

1. Serinproteasevariante, die eine Aminosäuresequenz mit einer Sequenzidentität von mindestens 80 % und weniger als 100 % mit der Aminosäuresequenz mit der SEQ ID NR: 31 aufweist, wobei die Aminosäure, die der 12. Position der Aminosäuresequenz mit der SEQ ID NR: 31 entspricht, mit einer unterschiedlichen Aminosäure substituiert ist und wobei die Serinproteasevariante eine Enzymaktivität aufweist, die, verglichen mit dem Protein, das die Serinproteaseaktivität vor einer Mutation aufweist, 100 % überschreitet.

2. Serinproteasevariante nach Anspruch 1, wobei die Aminosäure, die der 116. Position der Aminosäuresequenz mit der SEQ ID NR: 31 entspricht, ferner mit einer unterschiedlichen Aminosäure substituiert ist.

3. Serinproteasevariante nach Anspruch 1, wobei die Aminosäure, die der 12. Position entspricht, mit Tyrosin (Y), Alanin (A), Serin (S) oder Arginin (R) substituiert ist.

4. Serinproteasevariante nach Anspruch 2, wobei die Aminosäure, die der 116. Position entspricht, mit Aspartat (D), Serin (S), Threonin (T) oder Glycin (G) substituiert ist.

5. Serinproteasevariante nach Anspruch 1 oder 2, die eine Sequenzidentität von mindestens 80 % und weniger als 100 % mit der SEQ ID NR: 2 aufweist.

6. Serinproteasevariante nach Anspruch 1 oder 2, wobei die Serinproteasevariante eine Aminosäuresequenz aufweist, wie in einer beliebigen dargelegt, die ausgewählt ist aus der Gruppe, die besteht aus den SEQ ID NR: 3 bis 10 und den SEQ ID NR: 32 bis 39.

7. Polynukleotid, das die Serinproteasevariante nach einem der Ansprüche 1 bis 6 codiert.

8. Vektor, der das Polynukleotid nach Anspruch 7 umfasst.

9. Mikroorganismus, der die Serinproteasevariante nach einem der Ansprüche 1 bis 6 exprimiert.

10. Mikroorganismus nach Anspruch 9, wobei der Mikroorganismus zur Gattung *Bacillus* gehört.

11. Mikroorganismus nach Anspruch 10, wobei der Mikroorganismus *Bacillus subtilis* ist.

12. Futterzusammensetzung, welche die Serinproteasevariante nach Anspruch 1 oder 2 und/oder einen Mikroorganismus umfasst, der diese exprimiert.

## Revendications

1. Variant de sérine protéase comprenant une séquence d'acides aminés ayant une identité de séquence d'au moins 80 % et inférieure à 100 % avec la séquence d'acides aminés de SEQ ID NO: 31, dans lequel l'acide aminé correspondant à la 12^{ème} position de la séquence d'acides aminés de SEQ ID NO: 31 est substitué par un acide aminé différent et où ledit variant de sérine protéase a une activité enzymatique supérieure à 100 % par rapport à la protéine ayant l'activité de sérine protéase avant mutation.

2. Variant de sérine protéase selon la revendication 1, dans lequel l'acide aminé correspondant à la 116^{ème} position de la séquence d'acides aminés de SEQ ID NO: 31 est en outre substitué par un acide aminé différent.

3. Variant de sérine protéase selon la revendication 1, dans lequel l'acide aminé correspondant à la 12^{ème} position est substitué par de la tyrosine (Y), de l'alanine (A), de la sérine (S) ou de l'arginine (R).

4. Variant de sérine protéase selon la revendication 2, dans lequel l'acide aminé correspondant à la 116^{ème} position est substitué par de l'aspartate (D), de la sérine (S), de la thréonine (T) ou de la glycine (G).

5. Variant de sérine protéase selon la revendication 1 ou 2, qui présente une identité de séquence avec SEQ ID NO: 2 d'au moins 80 % et inférieure à 100 %.

6. Variant de sérine protéase selon la revendication 1 ou 2, où le variant de sérine protéase comprend une séquence d'acides aminés choisi parmi l'une quelconque dans le groupe constitué par les SEQ ID NOs: 3 à 10 et les SEQ ID NOs: 32 à 39.

7. Polynucléotide codant pour le variant de sérine protéase selon l'une quelconque des revendications 1 à 6.

8. Vecteur comprenant le polynucléotide selon la revendication 7.

9. Microorganisme exprimant le variant de sérine protéase selon l'une quelconque des revendications 1 à 6.

10. Micro-organisme selon la revendication 9, dans lequel le micro-organisme appartient au genre *Bacillus.*

11. Microorganisme selon la revendication 10, dans lequel le microorganisme est *Bacillus subtilis.*

12. Composition alimentaire comprenant le variant de sérine protéase selon la revendication 1 ou 2 et/ou un micro-organisme exprimant celui-ci.
